# EUROPEAN PATENT APPLICATION

(11) **EP 4 053 259 A1**
(43) Date of publication of application: **07.09.2022**
(21) Application number: 21305251.7
(22) Date of filing: 02.03.2021
(51) Int. Cl.: C12M 1/00, G02B 6/032

(54) **INTERNALLY ILLUMINATED PHOTO BIOREACTOR WITH LIGHT PIPE DISTRIBUTOR FOR PHOTO-REACTIVE MICROORGANISM CULTURE**

(71) Applicant: Syctom L'Agence Metropolitaine Des Dechets Menagers, 75013 Paris (FR); Setec Energie Environnement, 75583 Paris (FR); La Corporation de l'Ecole Polytechnique de Montréal, Montréal, Québec H3T 1J4 (CA); ASSOCIATION POUR LA RECHERCHE DEVELOPPEMENT DES METHODES ET PROCESSUS INDUSTRIELS - A.R.M.I.N.E.S., 75272 Paris Cedex 06 (FR); Kungliga Tekniska Högskolan, 10044 Stockholm (SE)
(72) Inventor: EL BAHRAOUI, Naoufel, 900200 Tanger (MA); CHIDAMI, Saad, Longueuil, J4M 1G6, QC (CA); CHAOUKI, Jamal, Dorval, H9S3L4, QC (CA); ACIEN FERNANDEZ, Francisco Gabriel, 04120, Almería (ES)
(74) Representative: Plasseraud IP

(57) **Abstract**

The invention pertains to the field of CO2 capture and culture of photo-reactive microorganism. The invention relates to a photo-bioreactor (PBR) comprising photo-reactive microorganism being illuminated by internal light pipe distributor (LPD). The present invention also relates to a method for capturing CO2 and producing photo-reactive microorganism. The present invention finally relates to the transformation of the photo-reactive microorganism obtained by the present method or within the photo-bioreactor.

## Description

The invention pertains to the field of CO₂ capture and culture of photoreactive microorganism. The invention relates to a photo-bioreactor (PBR) comprising photo-reactive microorganism being illuminated by internal light pipe distributor (LPD). The present invention also relates to a method for capturing CO₂ and producing photo-reactive microorganism. The present invention finally relates to the transformation of the photo-reactive microorganism obtained by the present method or within the photo-bioreactor.

### Technical Field

How to reduce CO₂ emissions and to utilize captured CO₂ are issues of main interests in the academic and industrial fields. CO₂ capture, sequestration, and utilization strategies adopted so far can be broadly divided into physical and biological means. Physical methods including capturing, transporting and storing CO₂ are very expensive processes. Biological utilization of CO₂ emission by photoreactive microorganism culture proves to be a promising alternative to physical methods. Photo-reactive microorganism culture can convert CO₂ through photosynthesis into organic chemicals such as carbohydrates, proteins, lipids, and other high added-value biofuels, which cannot only help mitigate global warming effects due to CO₂ emission, but can also alleviate the energy crisis.

Currently, photoautotrophic culture is the only technically and economically feasible way to produce photo-reactive microorganism biomass at commercial scale. Two main types of photo-reactive microorganism farming systems can be distinguished: open systems (the cultivated photo-reactive microorganism are in contact with the open air), and closed system, for example in photo-bioreactors (PBRs) (the cultivated photo-reactive microorganism are in a closed container).

Open systems are difficult to control because the parameters such as light availability, mixing, pH, temperature and nutrient concentration cannot be easily controlled. In contrast, closed PBRs allow great and diversified control of the parameters.

Either in the open or closed systems, light availability proves to be the most limiting factor for photo-reactive microorganism growth.

One major issue in photo-reactive microorganism culture is, thus, to provide sufficient and uniform quantity of light to each photo-reactive microorganism because the light is quickly absorbed by the surface-exposed photo-reactive microorganism cells. The higher the concentration of photo-reactive microorganism, the more light is absorbed. As a result, photo-reactive microorganism cells closed to the light source receive far more light than photoreactive microorganism in darker regions. The photo-reactive microorganism in the darker regions cannot get sufficient light for growth.

One could think about increasing the light intensity so as to decrease the volume of darker regions. However, this solution should be avoided because photo-reactive microorganism are limited in photosynthesis and oversaturated illumination can lead to irreversible damage due to photo-inhibition effects.

One could therefore think of drastically limiting the depth of the PBR to several millimeters to centimeters. Such limitation leads to very thin and long reactors, and, in consequence bulky system which requires large areas to be operated industrially. Moreover, gas transfer and mixing in such thin and long reactors is insufficient, which decreases the efficiency of the photo-reactive microorganism production.

There are two main ways to enhance the light distribution inside a photoreactive microorganism farming. One way consists in increasing the mixing of the photo-reactive microorganism inside the system. The mixing enhances the photoreactive microorganism cell circulation between the illuminated and dark regions. Such a mixing can be done for example by means of gas bubbling, installation of stirrers and mixers. However, inherent sensitivity of photo-reactive microorganism to strong shear stresses has limited further improvement on photo-reactive microorganism production by using the mixing solution.

Another way to enhance the light distribution inside a photo-reactive microorganism farming consists in bringing light inside the PBR as close as possible to the photo-reactive microorganism. By internally illuminating the photoreactive microorganism, the light is not delivered via the reactor surface by light-emitting structures outside the reactor, but via light-emitting structures disposed directly inside the reactor. The reactor geometry is not limited to very small layer thickness unlike the externally illuminated PBRs, and can thus be designed to be easily scaled up to industrial application.

Internally illuminated PBRs (IIPBRs) can be divided into two main categories: PBRs with internal light guides and PBRs with internal light sources.

PBRs with internal light sources have light sources inside the PBRs itself. Internal illumination via internal light sources leads to much lower light losses but higher volume losses. However, the heat released by internal light sources will result in temperature rise of the culture medium (21) and even cause damages to the photo-reactive microorganism cells.

PBRs with internal light guides comprise an external light source and light guides coupled to the external light source and transmitting the light from the outside into the reactor. For example, types of light guides can either consist of optical fibers or optical plates. Such a PBR has low volume losses since the volume of a light source is larger than the volume of a light guide, but substantial light losses will occur during light guiding process. Another issue is that the scaleup and the following maintenance process may be expensive and laborious.

### Technical Problem

As aforementioned, there is a plurality of systems for producing photoreactive microorganism. However, open systems are difficult to control and are subject to technical issues relating to the mixing of gas with the photo-reactive microorganism. Close systems are more easily operated. However, photo-reactive microorganism need to be consistently and sufficiently illuminated without being overheated or over-illuminated. Additionally, close systems for large scale production of photo-reactive microorganism still need to be developed.

There is therefore still a need for developing a photo-reactive microorganism farming enabling large-scale production of photo-reactive microorganism with low energy consumption, being cost-effective and being less bulky than large open system.

Also, there is still a need for developing a system for illuminating photoreactive microorganism with sufficient amount of light and uniformly.

### Summary of the invention

The invention is therefore directed to a light pipe distributor comprising a closed tubular external structure, a liquid filling the closed tubular external structure and a light inlet, wherein the refraction index of the liquid and the refraction index of the material of the closed tubular external structure are chosen so that light input through the light inlet is uniformly distributed through the liquid and all over the closed tubular external structure.

The closed tubular external structure may be made of two walls which are parallel to each other.

The two walls may be made of different materials or a same material.

Each of the walls of the closed tubular external structure may be made of material chosen from the list consisting of poly(methyl methacrylate), fluorinated ethylene propylene and polycarbonate.

The liquid may be chosen in the list consisting of water, alcohol.

The liquid may preferably be water further comprising an antimicrobial compound.

According to another embodiment, the invention is directed to a photo-bioreactor comprising:
- a tank with a gas inlet (50) fluidly connected to at least one nozzle, and a culture volume comprising a culture medium (21) and at least one species of photoreactive microorganism;
- a light pipe distributor according to one of claim 1 to 6;
- a light source external to the tank coupled to the light inlet of the light pipe distributor.

The closed tank may have a cylindrical shape with a longitudinal axis and wherein the light pipe distributor may have a cylindrical shape with a longitudinal axis, the longitudinal axis of the tank and the longitudinal axis of the light pipe distributor being collinear.

The photo-bioreactor may further have a plurality of light pipe distributors as afore described and homogeneously arranged in the closed tank, for example their cross-sections form together circles which are concentric with the center of the cross-section of the closed tank, or their cross-sections are distributed in a triangular or squared pattern.

The at least one nozzle may be at the bottom of the tank.

Each light pipe distributor may have one light inlet at both ends thereof and each ligh inlet is couplable to a light source external to the tank.

The light source may be chosen from the group consisting of a LED source, a laser source, a xenon lamp, and a halogen lamp, preferably a LED source.

The culture medium may be chosen from the group consisting of river water, Arnon medium culture and a combination thereof

The photo-reactive microorganism may be chosen from bacteria or micro-algae. The photo-reactive microorganism is preferably a microalga that may be chosen from the group consisting of the following genera: *Botryococcus, Chlorella, Phaeodactylum, Scenedesmus, Synechocystis, Ankistrodesmus, Scenedesmus, Synechocystis, Anabaena, Spirulina, Nostoc, and Calothrix,* preferably *Scenedesmus.*

According to another embodiment, the invention is directed to a method for producing photo-reactive microorganism with a photo-bioreactor of the present disclosure, comprising:
- connecting the gas inlet of the photo-bioreactor to an industrial chimney,
- illuminating the photo-reactive microorganism via the light pipe distributors,
- collecting the cultivated photo-reactive microorganism when the photo-reactive microorganism concentration in the PBR reaches a predetermined value.

The concentration of the photo-reactive microorganism inside the tank may be maintained between 0.2 and 10 g/L, preferably 1 and 5 g/L.

Finally, the invention is directed to a system comprising a photo-bioreactor as presently described and an industrial facility comprising an exhaust chimney, wherein the gas inlet of the photo-bioreactor is coupled to the exhaust chimney.

### Brief description of drawings

Other features, details and advantages will be shown in the following detailed description and on the illustrative figures, on which:
**Fig. 1**

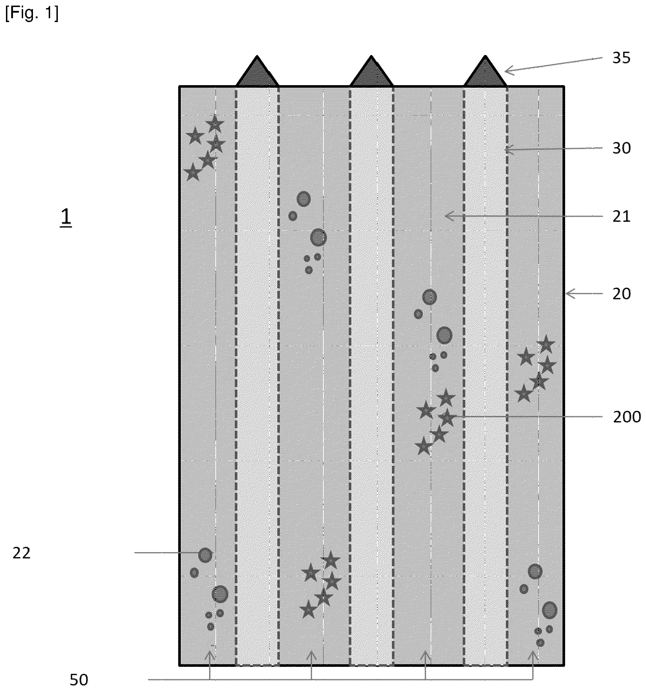

   [Fig. 1] is a longitudinal section of a PBR according to the invention, the different elements not being true to scale;
**Fig. 2**

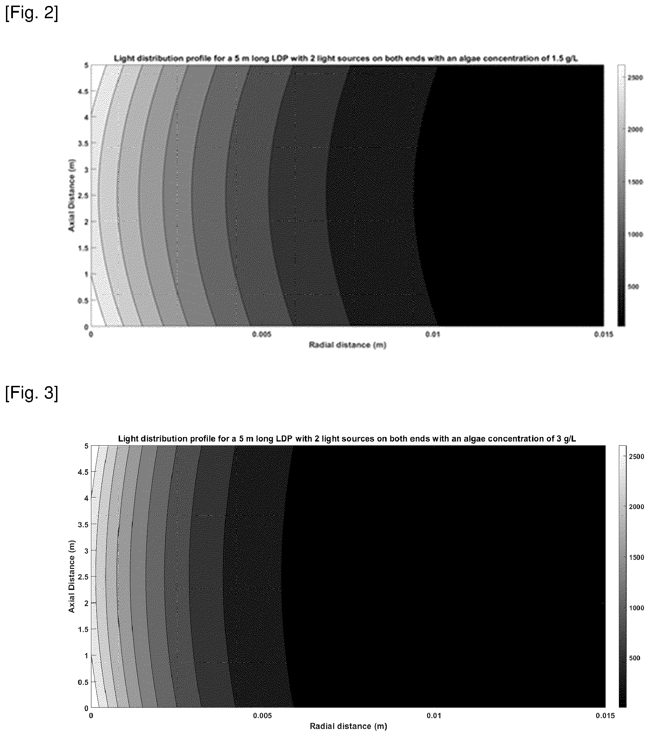

   [Fig. 2] is a graph of a cross-section of the PBR Light distribution profile for a 5 m long LDP with 2 light sources on both ends with a photoreactive concentration of 1,5 g/L;
**Fig. 3**
   [Fig. 3] is a graph of a cross-section of the PBR Light distribution profile for a 5 m long LDP with 2 light sources on both ends with a photo-reactive concentration of 3 g/L;
**Fig. 4**

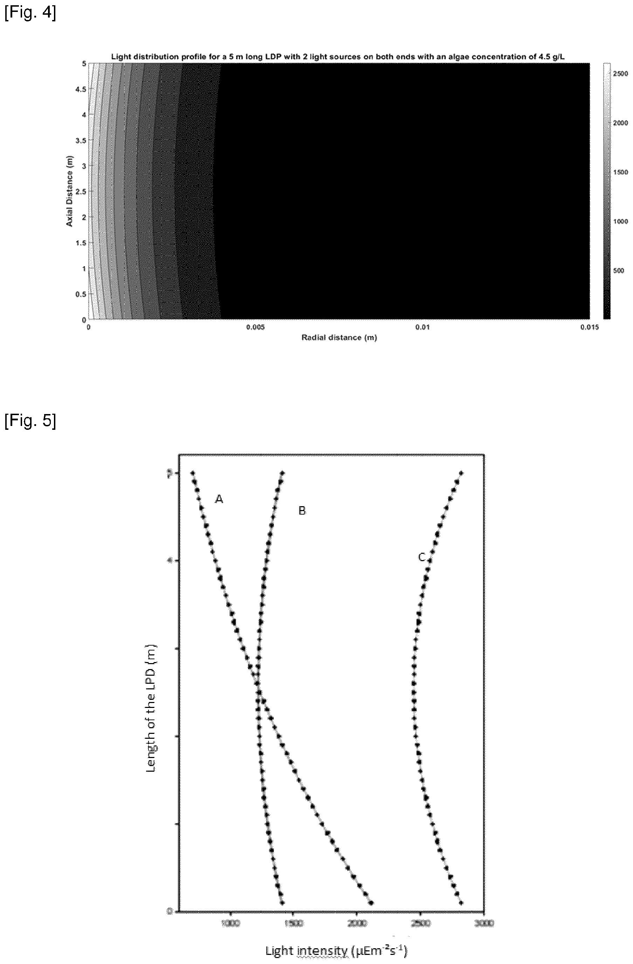

   [Fig. 4] is a graph of a cross-section of the PBR Light distribution profile for a 5 m long LDP with 2 light sources on both ends with a photo-reactive concentration of 4,5 g/L;
**Fig. 5**
   [Fig. 5] is a graph showing light intensity along the length of the LPD 30 when illuminated (A) from one end only at 100 % of the power of the light source, (B) from both ends at 50 % of the power of the light source, and (C) from both ends at 100 % of the power of the light source.
**Fig. 6**

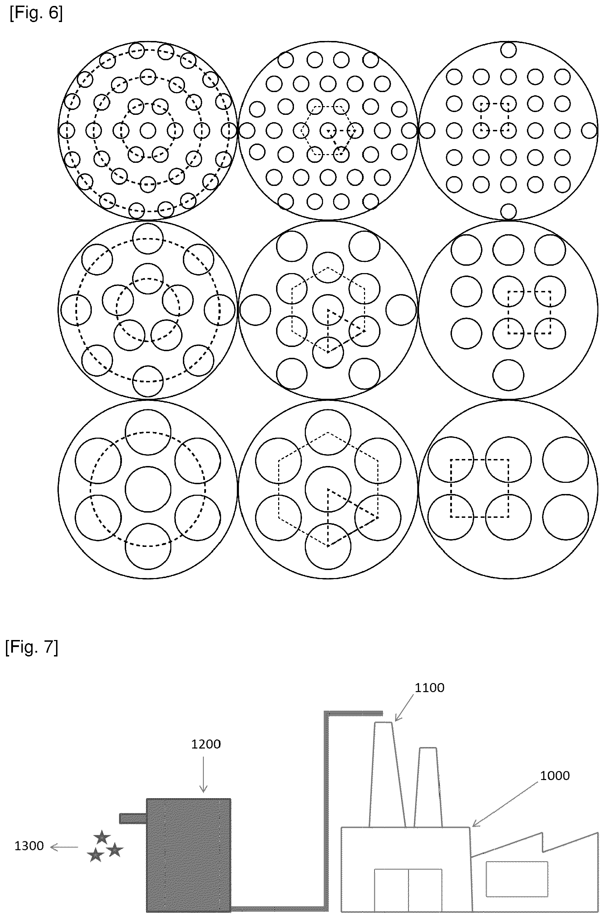

   [Fig. 6] is a cross-sectional view of different configurations of the LPDs in the PBR.
**Fig. 7**
   [Fig. 7] is a scheme of a photo bioreactor 1200 coupled to the chimney 1100 of an industrial facility.

### Description of embodiments

### Light pipe distributor 30 (LPD)

According to an object of the present invention, the inventors have developed a light pipe distributor 30 comprising a closed tubular external structure, a liquid filling the closed tubular external structure and a light inlet, wherein the refraction index of the liquid and the refraction index of the material of the closed tubular external structure are chosen so that light input through the light inlet is uniformly distributed through the liquid and all over the closed tubular external structure.

The light pipe distributor 30 addresses the problems of substantial light losses and heat generation inside a PBR. Such a LPD 30 is cost effective, easy to manufacture and to clean.

Refraction indexes of the closed tubular external structure and of the liquid are configured to uniformly distribute the light all over the closed tubular external structure toward the photo-reactive microorganism 200. The light coming through the light inlet of the LPD 30 is directed by the liquid all along the closed tubular external structure of the LPD 30 though reflection at the interface between the liquid and the closed tubular external structure. Also, every time a light ray reaches the interface, part of the light can be transmitted radially so that all of the photoreactive microorganism 200 receive sufficient light, whatever their position in the culture chamber of a photo bioreactor 1 in which the LPD 30 is installed.

Total internal reflection (TIR) is the phenomenon which occurs when a propagated wave strikes a medium boundary at an angle larger than a particular critical angle (TIR angle) with respect to the normal to the surface. TIR effects only occur when the light is transmitted from a denser medium, with a larger refractive index, to a thinner medium, with smaller refractive index.

It has been found that the larger the difference of refractive indexes between two adjacent media, the smaller the critical angle for the TIR phenomenon. A compromise between TIR effect and the transmission value of the closed tubular external structure has to be found. Indeed, if the TIR angle is too low, for example lower than about 30°, most of the light will stay inside the closed tubular external structure. In contrast, if the TIR angle is too high, for example above about 70°, most of the light coming from the light inlet will be transmitted through the part of the closed tubular external structure closest to the light inlet.

Thus, two characteristics have a significant impact on the light intensity and uniformity in an LPD 30: The first one is the amount of light staying inside the LDP due to the TIR phenomenon, which will determine the light uniformity and also the light intensity inside the LPD 30. The second one is the light transmission through the closed tubular external structure of the LDP which will determine the outside light intensity. The TIR phenomenon and light transmission characteristics are controlled by the dimension of the closed tubular external structure and the combination of the refraction indexes of the materials used for the closed tubular external structure and the liquid.

One user could adjust the refraction index of the liquid by directly modifying it, for example by adding of one or more additives having optical properties in the liquid or by changing the liquid itself. Additionally, one user may adjust the refraction index of the closed tubular external structure by modifying its geometry and/or its material.

The closed tubular external structure is for example a tube of plastic which is configured to be filled with the liquid.

The longitudinal section of each of the outer and inner surfaces of the tube forms a pattern. The pattern may be a straight line, sinusoidal line, serrated line or a line made from a random variation between two maxima; the line extending collinearly with the longitudinal axis of the tube. The lines formed by both the outer and inner surfaces may be in phase (maxima of the outer surface are at the same level as the maxima of the inner surface and minima of the outer surface are at the same level as the minima of the inner surface) or in opposite phase (the maxima of the outer surface are at the same level as minima of the inner surface and *vice versa*)*.* More generally, the lines of the outer and inner surfaces may be offset from one another. Such pattern may enhance the radial distribution of light toward the culture chamber.

The closed tubular external structure is then obtained by a rotation of the lines about the longitudinal axis of the tube.

If the pattern is a line, the closed tubular external structure is a right cylinder.

The closed tubular external structure may also be a tube comprising a horizontal cut which defines a polygon, for example a hexagon. In this case, the closed tubular external structure comprises facets that may enable the distribution of the light.

The closed tubular external structure may also comprise protrusions randomly distributed all over its surface or homogeneously arranged. The protrusions protrude from the outside of the closed tubular external structure. That is to say that the protrusions are within the tank, in contact with the culture medium and the photo-reactive microorganism. The protrusion may enhance the mixing of the photo-reactive microorganism and, if present, the bubbles. The protrusions may therefore decrease the time spend by the photo-reactive microorganism in darker regions.

The closed tubular external structure may be made of one or more walls.

In one example, the closed tubular external structure is made of a single wall. The single wall can be made of a material independently chosen from the list consisting of poly(methyl methacrylate) (PMMA), fluorinated ethylene propylene, and polycarbonate (PC). Preferably, the single wall is made of PC.

To adjust the refraction index, the closed tubular external structure may be made of two walls which are coaxial to each other.

To adjust the index refraction of a closed tubular external structure comprising two walls, the two walls may be fixed with each other or vacuum may be made between the two walls.

Preferably, the two walls are made of different materials or a same material.

Preferably, each of the walls of the closed tubular external structure is made of a material independently chosen from the list consisting of poly(methyl methacrylate) (PMMA), fluorinated ethylene propylene (FEP) and polycarbonate (PC), preferably PMMA.

Such materials reduce adhesion of the photo-reactive microorganism 200 onto the outer surface of the closed tubular external structure avoiding proliferation of the photo-reactive microorganism 200 thereon which would prevent the light from reaching the culture chamber. It makes it also easier to clean the outer surface of the closed tubular external structure.

The length of the cylinder is configured for industrial culture of photoreactive microorganism 200. The volume of the closed tubular external structure is preferably comprised between 0,05 m³ and 5 m³, preferably between 0,5 and 1 m³.

The closed tubular external structure and the liquid are preferably chosen so that the assembly they form are not configured to float in water or medium for cultivating photo-reactive microorganism 200. The material is preferably nontoxic for the photo-reactive microorganism 200 culture.

Additionally, one or more coatings or films can be applied to the wall(s) of the closed tubular external structure to provide it with further properties. For example, a light reflective coating or film can be applied to at least part or whole of a wall of the closed tubular external structure. It is also possible to apply such coatings or films to all walls of the closed tubular external structure. The same or different coatings or films can be applied to the walls.

The LPD 30 comprises a light inlet that makes the closed tubular external structure coupable to an external light source.

The light inlet may be a single opening at one end of the closed tubular external structure. The light inlet may be an opening made on a side of the closed tubular external structure. The light inlet mays also be an opening part of the plane perpendicular to the longitudinal axis of the closed tubular external structure, while being at its outermost end.

The light inlet may also comprise lens or optical system that enables to concentrate the light from an external source of light 35 into the interior of the closed tubular external structure.

The light inlet may also have a connector that would allow an external source of light 35 to be attached into it, for example by screwing or clipping.

The closed tubular external structure may be closed at one end and comprises a light inlet at the other end.

If the concentration of photo-reactive microorganism 200 is too high, it may be advantageous to use two external light sources per LPD, each coupled to one ends of the LPD 30. Figure 5 is a graph showing the light distribution profile for a 5m long LPD 30 with one single external light source 35 being switched on at 100% (curve A), with two external light sources being switched on at 50% (curve B), and two external light sources being switch on at 100% (curve C). The intensity is expressed in µE.m⁻².s⁻¹, measured at the outer surface of the closed tubular external structure of the LPD 30.

Thus, the closed tubular external structure may have two light inlets: one at each end of the closed tubular external structure. Preferably, each LPD 30 has one light inlet at both ends thereof and each light inlet is couplable to one or more light sources external to the tank 20.

Curve A has a minimum value of 200 µE.m⁻².s⁻¹ at the end with the light source turned off, and a maximum value of 2200 µE.m⁻².s⁻¹ at the end with the light source turned on. The decrease from the maximum value to the minimum value is not linear but is able to deliver a predetermined amount of light necessary for the growth of the photo-reactive microorganism.

Curve B has a minimum value of approximately 1300 µE.m⁻².s⁻¹ at the middle of the length of the closed tubular external structure, and a maximum value of approximately 1400 µE.m⁻².s⁻¹ at both ends thereof.

As it can be seen in figure 5, when both external light sources are switch on, the light at the ends of the closed tubular external structure may reach almost 3000 µE.m⁻².s⁻¹. At the middle of the closed tubular external structure, the light reaches almost 2700 µE.m⁻².s⁻¹.

A light pipe distributor 30 that has two or more external sources may decrease the amount of light needed at each end of the closed tubular external structure. Indeed, the power of the light source needs not be two high in order for the light to reach all the length of the LPD when there is a light inlet provided at both ends of thereof. If only one light inlet is provided, in order for the light to reach the other end of the LPD, the power of the external light source must be increase, which increases heat generation at the end where the light inlet is provided.

The LPD 30 may also comprise a light sensor to sense the amount of light inside the LPD 30. The LPD 30 may also comprise a light sensor movable inside the closed tubular external structure. The LPD 30 may comprise a light sensor fixed to the external surface of the closed tubular external structure.

The closed tubular external structure may be fully or partially filled with a liquid. The liquid transmits the light along the longitudinal axis of the LPD 30.

Preferably, the liquid inside the closed tubular external structure is chosen in the list consisting of water, alcohol. When the liquid is water, it is preferably deionized water.

Liquid is advantageously chosen regarding its refraction index and considering the refraction index of the closed tubular external structure.

Preferably, the liquid is water and further comprises an antimicrobial compound.

The liquid is also chosen so that it is inert to the material of the closed tubular external structure. Also, the liquid is preferably germ-free and in particular free of germs that can color the liquid and/or absorb a portion of the light propagating through the liquid.

Additionally, to adjust the refraction index of the liquid, the liquid may comprise inert particles, such as metallic particles, homogeneously distributed in the liquid. In such case, the LPD 30 may further comprise a mixer for mixing the liquid with metallic particles inside the external closed structure, so that distribution of the particles is even through out the external closed structure and the distribution of the light is enhanced.

### Photo-bioreactor (PBR)

According to another object of the present invention, the inventors have developed a photo-bioreactor comprising:
- a tank 20 with a gas inlet 50 and a culture volume comprising a culture medium 21 and at least one species of photo-reactive microorganism 200;
- a LPD 30 as described above; and
- a light source external to the tank 20 coupled to the light inlet of the light pipe distributor 30.

The inventors have demonstrated that such a PBR can reach an average light intensity of at least 100 µEm⁻²s⁻¹ to 2000 µEm⁻²s⁻¹, with a biomass concentration of 1 g/L to 5 g/L. Such results show that the PBR of the present invention can be used for an industrial production of photo-reactive microorganism.

Figure 1 is a longitudinal view of a photo-bioreactor according to one embodiment of the present invention. The PBR 1 comprises a tank 20, a light pipe distributor 30 and an external light source 35. The tank 20 is full of a culture medium 21. The culture medium 21 is a specific medium that enables the photo-reactive microorganism 200 to growth.

### LPD 30

The length of the LPD 30 may be the same as the one of the tank 20. For example, the tank 20 and the closed tubular external structure have a length of at least 3m.

The LPD 30 may be fully immerged in the tank 20 or partially immerged. Preferably, the closed tubular external structure of the LPD 30 is fully immerged so that the upper surface of the culture medium 21 in the tank 20 almost reaches the upper surface of the closed tubular external structure. In such an embodiment, the external source of light 35 is distant to the culture medium 21. Heat produced by the external source of light 35 is not conducted into the culture medium 21.

Preferably, the PBR has a plurality of light pipe distributors 30 homogeneously arranged in the closed tank 20 (notably their longitudinal axes are all parallel to one another and to the longitudinal axes of the tank 20), for example their cross-sections form together circles which are concentric with the center of the cross-section of the closed tank 20, or their cross-sections are distributed in a triangular or squared pattern.

Figure 6 is a cross-sectional view of different configurations of LPDs 30 in the PBR 1. In figure 6, it can be seen LPDs homogeneously distributed in concentric circles, squares or triangular.

As can be seen in figure 6a, in one example, 7 LPDs are evenly distributed so that one LPD is at the center and six are evenly distributed in a circle concentric with the one at the center. The distribution of the LPDs has therefore a rotational symmetry of 60°. This example can also be seen as a homogenous triangular distribution (see figure 6d).

As can be seen in figure 6b, in another example, 13 LPDs are distributed so that 5 are evenly placed in a first smaller circle concentric with the center of the PBR and 8 are evenly placed in a second bigger circle concentric with the center of the PBR.

As can be seen in figure 6c, in still another example, there are 34 LPDs distributed over 3 circles concentric with each other and with an LPD placed at the center of the PBR. 6 LPD are evenly placed in a first circle, 12 LPD are evenly placed in a second circle greater than the first circle and 18 LPD are evenly placed in a third circle greater than the second circle. The radius of each circle is r₁ < r₂ = 2*n₁ < r₃ = 2*r₁.

Figures 6d to 6f show LPDs distributed in a triangular pattern. Figure 6d is the same as figure 6a. Figure 6e shows 13 LPDs distributed so that one is in the center, 6 LPDs surrounds the central one and 6 other LPDs surrounds the 6 LPDs. Figure 6f shows 37 LPDs distributed so that one is in the center. In each of these embodiments, each LPD is separated from its closest neighbors by a distance which is the same for all LPDs and their closest neighbors.

Figures 6g to 6i show examples of LPDs distributed in a squared pattern. In figure 6g, there are 6 LPDs distributed over two "rows" and three "columns". In figure 6h, there are 10 LPDs distributed over four "rows" and three "columns", the last row having only one LPD. Figure 6i shows 29 LPDs distributed over five rows and five columns to form a square and the middle row and middle column having two additional LPDs, one at each side of the square.

The number of LPD 30 may be chosen according to the capacity of the tank 20. Too many LPDs 30 would decrease the volume of cultivated photoreactive microorganism but enhance the amount of light in the culture medium 21. In contrast, too few LPD 30 would increase the volume of cultivated photo-reactive microorganism but decrease the uniformity of light all over the culture medium 21 because they would be too far from each other so that the decrease of light intensity of one LPD 30 cannot be compensated by the ligh intensity of another LPD 30.

Preferably, the ratio of the illuminating surface area of the at least one closed tubular external structure of the at least one LPD 30, over the culture volume is comprised between 2 and 15 m⁻¹ and preferably between 5 and 13 m⁻¹.

The LPDs may be independently operated. For example, when homogeneously distributed in circles (cf. Fig. 6) only LPD in the center may be operated while the LPDs on the edge are switch off.

### Tank 20

Preferably, the closed tank 20 has a cylindrical shape with a longitudinal axis and wherein the light pipe distributor 30 has a cylindrical shape with a longitudinal axis, the longitudinal axis of the tank 20 and the longitudinal axis of the light pipe distributor 30 being collinear.

However, the shape of the tank is absolutely not limited to a cylindrical shape. The tank may be a three dimension container defined by a base and a length. The base may be polygon, a circle or a random shape in order to optimise the mixing of the photo-reactive microorganisms within the tank.

The tank 20 may be open or closed. Preferably, the tank 20 is closed so that only light from the light pipe distributor 30 is distributed to the photo-reactive microorganism. A closed tank 20 enables one operator to easily operate the different parameters of the PBR, i.e. amount of light, mixing, temperature, pH.

If the tank 20 is closed, the tank 20 comprises a cover. The external source of light 35 may be fixed to the cover and be placed in order to face light inlet of the LPD 30.

Also, the cover may have holes of the same dimension of the one of the closed tubular external structure so that one extremity of the LPD 30 is outside of the inside of the tank 20.

The cover may be made in a heat conducting material so that heat from external light source is dissipated.

The cover may further comprise a radiator to enhance the heat dissipation;

If the tank 20 is open, apart from the possibility for the light inlet to be connected to an external artificial source of light, it is also possible to connect it with a light concentrator for concentrating natural light inside the LPD 30.

The tank 20 is preferably in a non-transparent material. The tank 20 may comprise a window made of a transparent material so that it is possible to have a look of the inside of the tank 20 while it is not needed to remove the cover.

The tank 20 is preferably made from metal or rigid plastic.

Additionally, the tank 20 may also comprise stirrers and mixers to increase the mixing of the photo-reactive microorganism.

### Gas inlet 50

The gas inlet 50 makes it possible to bring gas inside the tank 20, which is necessary for the photo-reactive microorganism's growth.

The tank 20 may further comprise at least one nozzle fluidly connected to the gas inlet 50. The nozzle enables to distribute gas from the gas inlet 50 to the photo-reactive microorganism.

The at least one nozzle is preferably placed at the bottom of the tank 20. It makes it possible to move about the photo-reactive microorganism in the tank 20 thanks to the bubbles of injected gas moving upward from the bottom of the tank 20 so that each cell of photo-reactive microorganism receives sufficient quantity of light.

Preferably, the gas inlet 50 comprises a plurality of gas nozzles uniformly distributed all over the bottom of the tank 20, so that the photo-reactive microorganism are uniformly mixed by the gas bubbles 22 inside the culture medium 21, as it can be seen in figure 6.

The gas inlet 50 may also comprise a regulator to be operated to increase or decrease the volume of gas. For example, it could be needed to increase the volume of gas when the photo-reactive microorganism concentration is high.

The gas inlet 50 may further comprise a valve that makes it possible to choose which nozzle to fluidly connect to the gas inlet 50.

### Light source

The light source is preferably chosen from the group consisting of a LED source, a laser source, a xenon lamp, and a halogen lamp, preferably a LED source.

The light source may also comprise a passive or active heat management system. An example of passive heat management systems is a radiator. An example of active heat management systems is an air cooler. Also, a fan can be used and placed close to the light source to cool them.

The external source of light 35 may also be connected to a light concentrator. One end of the concentrator is fixed to the external source of light 35 while the other end is fixed to the light inlet of the LPD 30.

The concentrator may be a funnel made from mirrors or metal sheets that guide the light from the external source of light 35 with one end of the closed tubular external structure though the light inlet.

The external light source may be distant from the light inlet so that the heat produces by the external source of light 35 is managed. For example, the concentrator may be sufficiently long so that the external source of light 35 and the upper surface of liquid are distanced of at least 5 cm.

### Culture medium 21

Preferably, the culture medium 21 is chosen from the group consisting of river water, Arnon medium culture and a combination thereof

Preferably, the photo-reactive microorganism is a micro-algae is chosen from the group consisting of the following genera: *Botryococcus, Chlorella, Phaeodactylum, Scenedesmus, Synechocystis, Ankistrodesmus, Scenedesmus, Synechocystis, Anabaena, Spirulina, Nostoc,* and *Calothrix,* preferably *Scenedesmus.*

The PBR may also comprise at least one light sensor placed inside the tank 20 in the culture medium 21, for example at a point of the horizontal plane passing through the middle of the tank 20. The light sensor may also be movable so that a plurality of zones inside the tank 20 may be measured.

The PBR may further comprise sensors for other parameters, such as temperature, pH or concentration of a specific chemical compound.

### Culture method

According to another object of the invention, the inventors have developed a method for producing photo-reactive microorganism with a photo-bioreactor of the present disclosure, comprising:
- connecting the gas inlet 50 of the photo-bioreactor to an industrial exhaust pipe,
- illuminating the photo-reactive microorganism through the light pipe distributors,
- collecting the cultivated photo-reactive microorganism when the photo-reactive microorganism concentration in the PBR reaches a predetermined value.

The industrial exhaust pipe may be a chimney 1100 of a waste incinerator. Preferably, the gas of the industrial exhaust pipe is a CO₂-rich gas.

Figure 7 is a scheme of a system comprising an industrial facility (1000), for example a waste incinerator, and a photo bioreactor 1200 coupled to the industrial chimney 1100 of the industrial facility 1000.

According to another object of the present disclosure, the inventors have developed a system comprising a photo-bioreactor and an industrial facility 1000 comprising an exhaust pipe or chimney 1100, wherein the gas inlet 50 of the photo-bioreactor is coupled to the exhaust pipe or chimney 1100.

The system has the advantage of using the exhaust fumes coming from the industrial chimney 1100. The system also has the advantage of cultivating high added value photo-reactive microorganism that can be used in a lot of different fields, such as bio energy.

The gas exhausted by the chimney 1100 may contain gas that is consumed by the photo-reactive microorganism, such as CO2. While the gas is treated and the bad gas for the atmosphere is consumed, the photo-reactive microorganism are cultivated and the concentration in the tank 20 increases. When the concentration of photo-reactive microorganism in the tank 20 reaches a predetermined value, the photo-reactive microorganism are collected and transformed, for example in bio energy. Thus, the photo-reactive microorganism collected thanks to the system of the present invention are of high added value.

The gas of the industrial exhaust pipe may be treated before it enters the PBR via the gas inlet 50. For example, the gas of the industrial exhaust pipe is doped with a gas compound useful for the photo-reactive microorganism culture. For example, the gas of the industrial exhaust pipe is doped with CO₂ before it enters the gas inlet 50.

The gas inlet 50 may be coupled to a valve. The valve enables to control the quantity of gas to be injected inside the tank 20. Also, the quantity of gas injected inside the tank 20 can be continuously controlled according to the concentration of the photo-reactive microorganism inside the tank 20.

The culture of the photo-reactive microorganism in the PBR may be a continuous culture. In that case, the concentration of the photo-reactive microorganism inside the tank 20 is maintained between 0.2 and 10 g/L, preferably between 1 and 5 g/L.

The culture of the photo-reactive microorganism may also be in batches. In that case, each time the concentration of the photo-reactive microorganism in the tank 20 reaches a predetermined value. The culture is stopped and the photoreactive microorganism are collected.

The illumination of the photo-reactive microorganism may be continuous or discontinuous. For example, the LPD 30 may be operated so that they illuminate the photo-reactive microorganism for a predetermined time and turns off for another predetermined time. Also, the way of illuminating the photo-reactive microorganism could follow a predetermined pattern of ON/OFF periods and of different light powers. For example, when the concentration of photo-reactive microorganism is low, the LPD 30 are operated at high low power for a predetermined time. Then, the light power increases while the concentration of photo-reactive microorganism increases.

Also, the concentration of photo-reactive microorganism in the tank 20 could be inhomogeneous. In that case, and if the tank 20 comprises a plurality of LPD 30, it could be advantageous to increase the illumination in the tank 20 where the concentration is high, or to decrease the illumination where the concentration is too low. Each of the external sources of light would be therefore operated according to the concentration of photo-reactive microorganism in the tank 20 closed to them.

When switched on, the external light source emits light that is conducted from the light inlet to the top and bottom of the LPD 30 through the liquid and all over the closed tubular external structure of the LPD 30. As the light went from the upper to the lower end of the LPD 30, the light is also radially distributed in the culture medium 21 to the photo-reactive microorganism 200.

The light source may be further configured to be operated in order to change its emission spectrum, thus making it possible to match the emission spectrum with the culture stage of the photo-reactive microorganism. For example, the light source spectrum is close to the solar spectrum, i.e. for each wavelength, the relative intensity (wavelength intensity over total intensity) is at least 85 % of the relative intensity of the solar spectrum. Also, the relative intensity of the light source in specific wavelength ranges can be adjusted.

Each external source of light 35 may be independently operated. For example, the concentration of photo-reactive microorganism in the lower part of the tank 20 can be higher than that in the upper part. In that case, it may be beneficial to enhance the illumination of the photo-reactive microorganism in the lower part in comparison with the upper part. Thus, the light power of the light external source is in the lower end of the LPD 30 can be increased.

The collected photo-reactive microorganism 1300 may be transformed in order to make bio plastic, bio energy, food supplements, fish or pet food, bio cosmetic, medicine or all of the potential use corresponding to the specific cultivated photo-reactive microorganism.

### Example

Figures 2, 3 and 4 are three graphs showing the propagation of light from the closed tubular external structure of a LPD in the culture medium for three photo-reactive microorganism concentrations in the tank: respectively 1.5 g/L, 3g/L and 4.5g/L.

According to this embodiment, the LPD is a cylinder of 5m, both ends of the cylinder have a source of light that makes the light profile in the culture medium homogeneous. The sources of light may be operated accordingly to the concentration of photo-reactive microorganism, the more the concentration of photo-reactive microorganism, the more power the external source of light get.

As it can be seen in figures 2, 3 and 4, the higher the concentration of photo-reactive microorganism in the tank, the lower the propagation of light in the culture medium is.

### Reference Signs List

- 1: Photo bioreactor - 20: tank - 21: culture medium - 22: gas bubble - 200: photo-reactive microorganism - 30: light pipe distributor - 35: external source of light - 50: gas inlet - 1000: industrial facility - 1100: industrial chimney - 1200: photo bioreactor - 1300: cultivated photo-reactive microorganism

## Claims

1. A light pipe distributor comprising a closed tubular external structure, a liquid filling the closed tubular external structure and a light inlet, wherein the refraction index of the liquid and the refraction index of the material of the closed tubular external structure are chosen so that light input through the light inlet is uniformly distributed through the liquid and all over the closed tubular external structure.

2. The light pipe distributor according to claim 1, wherein the closed tubular external structure is made of two walls which are parallel to each other.

3. The light pipe distributor according to claim 2, wherein the two walls are made of different materials or a same material.

4. The light pipe distributor according to claim 3, wherein each of the walls of the closed tubular external structure is made of material chosen from the list consisting of poly(methyl methacrylate), fluorinated ethylene propylene and polycarbonate.

5. A photo-bioreactor comprising:
- a tank with a gas inlet (50) fluidly connected to at least one nozzle, and a culture volume comprising a culture medium (21) and at least one species of photoreactive microorganism;
- a light pipe distributor according to one of claim 1 to 4;
- a light source external to the tank coupled to the light inlet of the light pipe distributor.

6. The photo-bioreactor according to claim 5, further having a plurality of light pipe distributors according to one of claims 1 to 4 and homogeneously arranged in the closed tank, for example their cross-sections form together circles which are concentric with the center of the cross-section of the closed tank, or their cross-sections are distributed in a triangular or squared pattern.

7. The photo-bioreactor according to any one of claims 5 to 6, wherein the at least one nozzle is at the bottom of the tank.

8. The photo-bioreactor according to any one of claims 5 to 7, wherein each light pipe distributor has one light inlet at both ends thereof and each light inlet is couplable to a light source external to the tank.

9. The photo-bioreactor according to any one of claims 5 to 8, wherein the light source is chosen from the group consisting of a LED source, a laser source, a xenon lamp, and a halogen lamp, preferably a LED source.

10. The photo-bioreactor according to any one of claims 5 to 9, wherein the culture medium (21) is chosen from the group consisting of river water, Arnon medium culture and a combination thereof

11. The photo-bioreactor according to any one of claims 5 to 10, wherein the photo-reactive microorganism is a micro-algae being chosen from the group consisting of the following genera: *Botryococcus, Chlorella, Phaeodactylum, Scenedesmus, Synechocystis, Ankistrodesmus, Scenedesmus, Synechocystis, Anabaena, Spirulina, Nostoc, and Calothrix,* preferably *Scenedesmus.*

12. A method for producing photo-reactive microorganism with a photo-bioreactor according to any one of claims 5 to 11, comprising:
- connecting the gas inlet (50) of the photo-bioreactor to an industrial chimney (1100),
- illuminating the photo-reactive microorganism via the light pipe distributors,
- collecting the cultivated photo-reactive microorganism when the photo-reactive microorganism concentration in the PBR reaches a predetermined value.

13. A method for producing photo-reactive microorganism with a photo-bioreactor according to claim 5 to 11, wherein a concentration of the photoreactive microorganism inside the tank is maintained between 0.2 and 10 g/L, preferably 1 and 5 g/L.

14. A system comprising a photo-bioreactor according to any one of claim 5 to 11 and an industrial facility comprising an exhaust chimney (1100), wherein the gas inlet (50) of the photo-bioreactor is coupled to the exhaust chimney (1100).
